# EUROPÄISCHE PATENTSCHRIFT

(11) **EP 3 004 044 B1**
(45) Veröffentlichungstag und Bekanntmachung des Hinweises auf die Patenterteilung: **19.04.2017**
(21) Anmeldenummer: 14721306.0
(22) Anmeldetag: 28.04.2014
(51) Int. Cl.: C07C 51/347

(54) **VERFAHREN ZUR DEHYDRATISIERUNG VON ALPHA-SUBSTITUIERTEN CARBONSÄUREN**
METHOD FOR DEHYDRATING ALPHA-SUBSTITUTED CARBOXYLIC ACIDS
PROCÉDÉ DE DÉSHYDRATATION D'ACIDES CARBOXYLIQUES ALPHA-SUBSTITUÉS

(30) Priorität: 27.05.2013 DE 102013209821
(43) Veröffentlichungstag der Anmeldung: 13.04.2016
(73) Patentinhaber: Evonik Röhm GmbH, 64293 Darmstadt (DE)
(72) Erfinder: MAY, Alexander, 64342 Seeheim-Jugenheim (DE); KRILL, Steffen, 64367 Mühltal (DE); BECKER, Jörg, 76135 Karlsruhe (DE); PLÖSSER, Willi, 64342 Seeheim-Jugenheim (DE); TRESKOW, Marcel, 64293 Darmstadt (DE); KÖSTNER, Martin, 64295 Darmstadt (DE)
(86) Internationale Anmeldenummer: PCT/EP2014/058578
(87) Internationale Veröffentlichungsnummer: WO 2014/191145

(56) Entgegenhaltungen:
- DE-A1- 1 768 253

## Beschreibung

Die vorliegende Erfindung beschreibt ein Verfahren zur Herstellung von alpha-beta-ungesättigten Carbonsäuren durch Dehydratisierung von in alpha-Stellung substituierten Hydroxy- oder Alkoxy-Carbonsäuren, insbesondere von 2-Hydroxyisobuttersäure (2-HIBS), unter Vermeidung der Akkumulation von Nebenprodukten und zugesetzter Additive.

Entsprechende Verfahren sind aus dem Stand der Technik hinlänglich bekannt. In CH 430691 wird die Dehydratisierung von in Methanol gelöster 2-HIBS in der Flüssigphase mit NaOH als Katalysator unter Bildung von MMA und MAS beschrieben. Der Katalysator wird nur in geringen Mengen zugegeben. Zum Erreichen der hohen Temperatur von 260 °C werden als Badflüssigkeit Phthalsäureanhydrid und Tetraethylenglykol-dimethylether eingesetzt.

Die DE1568948 offenbart eine Dehydratisierung von in Alkohol gelöster oder geschmolzener 2-HIBS in der Flüssigphase an basischen Verbindungen als Katalysator, die zur Bildung von Hydroxyisobutyratanionen führen, in Gegenwart von hochsiedenden, polaren Lösungsmitteln.

In der DE1211153 wird ähnlich wie vorgenannt eine Dehydratisierung von in Alkohol gelöster 2-HIBS bzw. von 2-Hydroxyisobuttersäuremethylester (2-HIBSM) in der Gasphase an verschiedenen Festbettkatalysatoren bei 250-400 °C offenbart. Als Katalysatoren werden u.a. geträgerte Phosphate, geträgerte Sulfate, Al₂O₃, ZnO, MoO₃-Al₂O₃, MgO, BPO₄ eingesetzt.

In DE1768253 offenbarte Katalysatoren sind Alkali- und Erdalkalisalze von 2-HIBS (Na, K, Li, Ca, Mg, Ba, Sr) eingesetzt als z.B. Hydroxide, Carbonate, Sulfite, Acetate oder Phosphate. Bevorzugte Durchführung der Dehydratisierung bei Atmosphärendruck und 210-225 °C unter Zusatz von Polymerisationsinhibitoren. Diese Offenlegung beschreibt auch eine kontinuierliche Zufuhr des Katalysators und eine teilweise Ausschleusung des Reaktorinhalts, um dort die Akkumulation von Katalysator und Nebenprodukten zu vermeiden. Eine Rückgewinnung des dadurch notwendigerweise ebenfalls mit ausgeschleusten Zielproduktes wird jedoch nicht beschrieben.

EP 487853 offenbart ein Verfahren zur Herstellung von Methacrylsäure (MAS), welches folgende Schritte umfasst: a) Herstellung von Acetocyanhydrin (ACH) aus Aceton und HCN, b) Gewinnung von Hydroxyisobuttersäureamid (HIBA)-Synthese durch ACH-Hydrolyse an MnO₂, c) homogen katalytische Umsetzung von HIBA zu HIBSM mit Methylformiat oder MeOH/CO unter Bildung von Formamid und d) Hydrolyse von HIBSM zu 2-HIBS mit anschließender Dehydratisierung zu MAS und H₂O. Der letzte Reaktionsschritt wird als kontinuierlich beschrieben mit Zufuhr von Stabilisatoren. Auf die dadurch sich im Langzeitbetrieb zwangsläufig ergebenden Schwierigkeiten durch Akkumulation von Nebenprodukten etc. wird nicht eingegangen.

Nach DE 1191367 erfolgt die Dehydratisierung von alpha-Hydroxy-Carbonsäuren in Gegenwart von Cu und Hydrochinon als Polymerisationsinhibitor sowie einer Mischung aus Alkalichloriden oder -bromiden und entsprechenden Halogenidsalzen von Zn, Sn, Fe, Pb als Katalysator bei Temperaturen von 185-195 °C. Ein kontinuierlicher Betrieb sowie eventuelle Probleme bei Rückführungen werden nicht beschrieben. Eigene Versuche zeigen, dass zum Einen durch den Einsatz von Halogensalzen als Katalysator auch alpha-halogenierte Reaktionsnebenprodukte entstehen, die entsprechend aufwändig wieder vom eigentlichen Zielprodukt abgetrennt werden müssen, zum Anderen erfordert der Einsatz halogenierter Verbindungen aufgrund ihrer korrosiven Wirkung die Verwendung entsprechend beständiger technischer Werkstoffe, was das Verfahren insgesamt verteuert.

Nach der DE 102005023975 wird die Dehydratisierung in Gegenwart mindestens eines Metallsalzes, beispielsweise von Alkali- und/oder Erdalkalimetallsalzen, bei Temperaturen von 160-300 °C, besonders bevorzugt von 200 bis 240 °C beschrieben. Zu den geeigneten Metallsalzen dort gehören u.a. Natriumhydroxid, Kaliumhydroxid, Calciumhydroxid, Bariumhydroxid, Magnesiumhydroxid, Natriumsulfit, Natriumcarbonat, Kaliumcarbonat, Strontiumcarbonat, Magnesiumcarbonat, Natriumbicarbonat, Natriumacetat, Kaliumacetat und Natriumdihydrogenphosphat. Als Besonderheit ist der Druck der Dehydratisierungsstufe gleich dem einer vorgeschalteten Umesterungstufe und liegt bevorzugt im Bereich 0,1-1 bar. Eine Ausschleusung von Nebenprodukten wird nicht offenbart.

Diesen im Stand der Technik beschriebenen, insbesondere als kontinuierliche Variante ausgelegten Verfahren ist gemein, dass zur Vermeidung von Polymerisation der Zielprodukte dem Reaktorfeed oder der Reaktionsmischungszufuhr in die in der Regel dem Reaktor nachgeschaltete Destillationskolonne Polymerisationsinhibitoren zugesetzt werden müssen. Ohne weitere Maßnahmen zur Abtrennung oder Ausschleusung reichern sich diese Substanzen im Reaktor, verbunden mit entsprechenden Nachteilen wie z.B. Belagsbildung oder Verfärbung, stetig an.

Ferner ist die Umsetzung der alpha-substituierten Carbonsäuren nie vollständig. Zum Erreichen eines wirtschaftlichen großtechnischen Verfahrens ist daher die Rückführung nicht umgesetzter Reaktanden unbedingt notwendig. Die im Stand der Technik zitierten Dokumente gehen weder auf eine solche notwendige Produktrückführung noch auf die Bildung und Beseitigung von Nebenprodukten ein. Hinzu kommt, dass unabhängig vom eingesetzten Katalysator bei der Umsetzung von z.B. alpha-Hydroxycarbonsäuren für diese Reaktion typische Nebenprodukte, die auch bei sehr guten Selektivitäten >98% stets zumindest in Spuren gebildet werden. All diesen Nebenprodukten ist gemein, dass sie einen Siedepunkt ähnlich der alpha-substituierten Carbonsäuren besitzen, und somit in der Rückführung der nicht umgesetzten Edukte zwangsweise akkumulieren. Gleiches gilt für der Reaktion zugesetzte Polymerisationsinhibitoren mit im Vergleich zum Edukt ähnlichen Siedepunkten.

Werden also keine weiteren technischen Massnahmen ergriffen, akkumulieren die beschriebenen Nebenkomponenten bzw. Additive in der Edukt-Rückführung kontinuierlich weiter, was zu einem Anstieg des Rückführungskreislaufstroms insgesamt führt und beim jeweiligen Wieder-Verdampfen im Reaktorkreislauf den Energieverbrauch erhöht. Zur Vermeidung dieser kontinuierlichen Akkumulation unerwünschter Nebenprodukte ist daher eine Ausschleusung erforderlich, mit der sich ein konstantes Nebenprodukt- bzw. Additivniveau im Reaktionssystem realisieren lässt. Wirtschaftlich gesehen ist eine undifferenzierte Ausschleusung jedoch nachteilig, da neben den unerwünschten Nebenprodukten und überschüssigen Polymerisationsinhibitoren auch Zielprodukt und Katalysator verloren gehen.

In der Veröffentlichung "Avoiding Accumulation of Trace Components" (Ind. Eng. Chem. Res. 1992, 31, 1502-1509) wird zwar in zahlreichen Varianten die mögliche Ausschleusung von in Spuren auftretenden Nebenprodukten diskutiert, allerdings wird nicht auf die Rückführung eventuell mit ausgeschleuster Zielprodukte nach dem Ausschleusungspunkt eingegangen.

Aufgabe der vorliegenden Erfindung ist es daher, zur Vermeidung der Anreicherung von unerwünschten Nebenprodukte und Additiven, insbesondere überschüssigen Polymerisationsinhibitoren, diese aus dem Reaktionsprozess zum Erreichen eines stabilen Konzentrationsgleichgewichtes auszuschleusen und dabei den Verlust an Zielprodukt zu minimieren und den Energieverbrauch zur Aufrechterhaltung der Kreislaufströme zu optimieren. Weitere nicht explizit genannte Aufgaben ergeben sich aus dem Gesamtzusammenhang der nachfolgenden Beschreibung, Ansprüche und Beispiele.

Gelöst wird diese Aufgabe durch ein Verfahren zur Dehydratisierung von alpha-substituierten Carbonsäuren dadurch gekennzeichnet, dass kontinuierlich zumindest ein Teil des Reaktionsstroms aus dem Reaktionskreislauf ausgeschleust, Nebenprodukte oder überschüssige Additive vom nicht umgesetzten Ausgangsprodukt abgetrennt werden und letzteres in den Reaktionskreislauf zurück geführt wird.

Als Edukte für dieses Verfahren eignen sich alpha-substituierte Carbonsäuren der Formel (1): 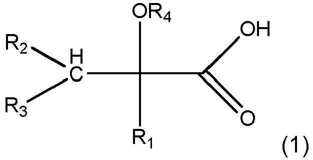 wobei R1 = H oder CHR'R"; R₂ bzw. R₃ unabhängig voneinander = H oder Kohlenstoffrest mit 1-7 Kohlenstoffatomen, linear, verzweigt oder alicylisch; R₄ = H oder Kohlenstoffrest mit 1-3 Kohlenstoffatomen, linear oder verzweigt; R' bzw. R" unabhängig voneinander H oder Kohlenstoffrest mit 1-3 Kohlenstoffatomen.

Bei den auszuschleusenden überschüssigen Additiven handelt es sich im Wesentlichen um Polymerisationsinhibitoren, die das Polymerisieren der als Zielprodukt entstehenden alpha-beta-ungesättigten Carbonsäuren verhindern sollen. Als Inhibitoren eignen sich beispielsweise Hydrochinone, Hydrochinonether, wie Hydrochinonmonomethylether oder Di-tert-butylbrenzcatechin, Phenothiazin, 4-Hydroxy-2,2,6,6-tetramethylpiperidin-1-oxyl, N,N'-Diphenyl-p-phenylendiamin, Methylenblau oder sterisch gehinderte Phenole (z.B. Topanol A), die in der Fachwelt weithin bekannt sind. Diese Verbindungen können einzeln oder in Form von Mischungen eingesetzt werden und sind im Allgemeinen kommerziell erhältlich. Die Wirkung der Stabilisatoren besteht meist darin, dass sie als Radikalfänger für die bei der ungewünschten Polymerisation auftretenden freien Radikale wirken. Für weitere Details und Alternativen wird auf die gängige Fachliteratur, insbesondere auf das Römpp-Lexikon Chemie; Herausgeber: J. Falbe, M. Regitz; Stuttgart, New York; 10. Auflage (1996); Stichwort "Antioxidantien" und die an dieser Stelle zitierten Literaturstellen verwiesen. Vorzugsweise werden insbesondere Phenole als Polymerisationsinhibitor eingesetzt. Besondere Vorteile können bei Verwendung von Hydrochinonmonomethylether erzielt werden. Bezogen auf das Gewicht der gesamten Reaktionsmischung kann der Anteil der Inhibitoren einzeln oder als Mischung im Allgemeinen 0,01-0,5 Gew% betragen und wird durch ein entsprechendes Dosierungs- und Ausschleusungsgleichgewicht in diesem Bereich gehalten.

Für die Dehydratisierungsreaktion typische Nebenprodukte sind z.B. dimere oder oligomere Formen der alpha-substituierten Hydroxycarbonsäuren, offenkettig oder als Ringverbindung, sowie Decarboxylierungsprodukte aus zwei dimeren alpha-beta-ungesättigten Carbonsäuren, im Wesentlichen sind dies Penten- (Formel (2)) oder Hexensäuren (Formel (3)): 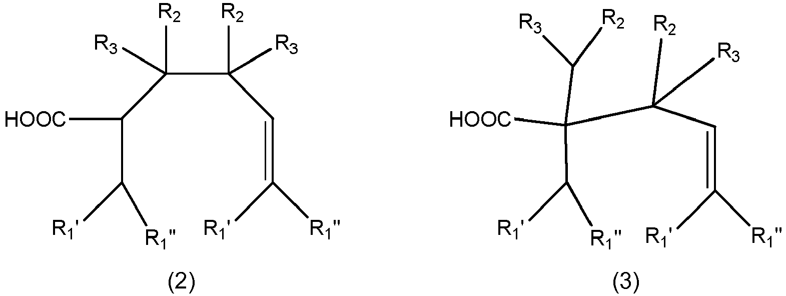 wobei alle R-Substituenten unabhängig voneinander H oder Kohlenstoffketten mit 1-3 Kohlenstoffatomen darstellen.

Erstere lassen sich durch geeignete Prozessführung wieder in die Ausgangsprodukte spalten, während letztere dauerhaft aus dem Prozess ausgeschleust werden müssen.

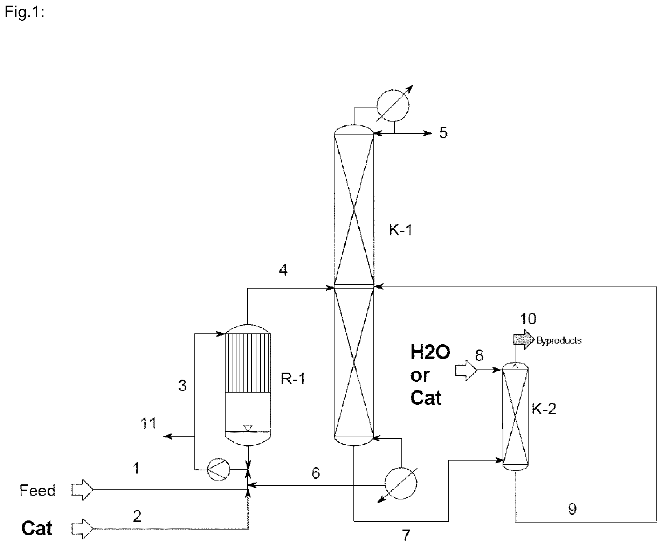

Eine bevorzugte Ausführungsform der Erfindung ist in Fig.1 dargestellt. Über eine Leitung 1 wird der Feed, die alpha-substituierte Hydroxy-Carbonsäure, als Schmelze, wässrige oder alkoholische Lösung dem Reaktorkreislauf R-1 zugeführt. Parallel kann über Leitung 2 der Katalysator kontinuierlich oder in Intervallen zugeführt werden. Der Katalysator kann dabei in Edukt, Produkt oder Wasser gelöst vorliegen. Das Edukt und der Katalysator werden durch den Kreislauf 3 über einen Wärmetauscherapparat R-1 geführt, bei dem es sich beispielsweise um einen Fallfilmwärmetauscher, Rohrbündelwärmetauscher mit oder ohne Einbauten in den Rohren zur Erhöhung der Turbulenz, wie sie z.B. von der Fal. Calgavin kommerziell erhältlich sind, oder einen Plattenwärmetauscher handeln kann. Am Wärmetauscher wird die für die Reaktion sowie das Verdampfen der Reaktionsprodukte notwendige Energie eingebracht. Der Abstrom des Reaktors wird direkt gasförmig über Leitung 4 einer Destillationskolonne K-1 zugeführt, in der Wasser sowie das Zielprodukt alpha-beta-ungesättigte Carbonsäure als Kopfprodukt über Leitung 5 abgetrennt werden. Die nicht umgesetzte Hydroxy-Carbonsäure wird am Kolonnensumpf über Leitung 6 als Teilstrom der Reaktion wieder zugeführt. Polymerisationsinhibitoren, die dem Feed und/oder der Kolonne K-1 zugeführt werden, sowie Nebenkomponenten, die den Reaktor R-1 gasförmig verlassen haben, jedoch in der Kolonne K-2 zusammen mit der Hydroxy-Carbonsäure in den Sumpf gelangen, werden ebenfalls über Leitung 6 rezykliert.

Ohne weitere technische Massnahmen akkumulieren sowohl diese Polymerisationsinhibitoren als auch die erwähnten Nebenprodukte im Reaktorkreislauf, was zu einer Volumenzunahme des Kreislaufstroms 6 selbst führt und beim erneuten Verdampfen der Reaktionsmischung in Reaktor R-1 den Energieverbrauch belastet. Um diese Nachteile zu vermeiden wird über eine Ausschleusung 7 ein Teilstrom des Kreislaufstroms 6 entnommen und einer Waschstufe K-2 zugeführt, der als Waschflüssigkeit 8 Wasser oder ein Teilstrom der wässrigen Katalysatorlösung zugeführt wird. Mögliche Apparteausführungen für die Waschstufe K-2 können sein: Mixer-Settler-Einheiten (einstufig oder mehrstufig, bevorzugt einstufig); statische, pulsierte oder gerührte Extraktionskolonnen. Typische Einbauten hierbei sind Füllkörper, Siebböden (statisch oder pulsierende Ausführung) sowie statische Packungen. Diese Apparateausführungen sind dem Fachmann bekannt, und u.a. beschrieben in K. Sattler, Thermische Trennverfahren, Wiley-VCH, 2001. Die im Strom 7 enthaltene Hydroxy-Carbonsäure geht dabei bevorzugt in die wässrige Phase, welche über Leitung 9 wieder zu Kolonne K-1 gelangt. Damit wird der Verlust an Zielprodukt minimiert. Die in Wasser unlöslichen Polymerisationsinhibitoren und Nebenprodukte bilden im Wäscher K-2 eine eigene organische Phase und können so über einen Auslaß 10 selektiv und vom Zielprodukt befreit aus dem Prozess ausgeschleust werden.

Sollten höhersiedende Nebenprodukte sich im Reaktorkreislauf 3 akkumulieren, die nicht über die Gasphase via Strom 4 in den Sumpf der Destillationskolonne K-1 gelangen können, besteht die Möglichkeit, diese direkt aus dem Reaktorkreislauf durch einen Teilstrom über Leitung 11 zu eliminieren. Die Ausschleusungsstrom 11 enthält dann im wesentlichen Katalysator und Hydroxy-Carbonsäure als wieder zu gewinnende Komponenten und kann zu diesem Zweck ebenfalls dem Wäscher K-2 zugeführt werden.

Die in der Waschkolonne K-2 durch Zuführung von Wasser oder wässriger Katalysatorlösung induzierte Phasentrennung verläuft überraschenderweise dann besonders gut, wenn die Konzentration an Nebenprodukten und/oder Polymerisationsinhibitoren im Ausschleusungsstrom 7 einen bestimmten Wert überschreitet. Es ist daher von Vorteil, den Ausschleusungsstrom 7 erst nach einer Induktionsperiode abzuzweigen, wenn diese Grenzkonzentration erreicht ist. Bei der Umsetzung von 2-HIBS zu MAS bedeutet dies eine Konzentration von 10-30 Gew%, bevorzugt 15-25 Gew%, besonders bevorzugt 17-22 Gew% 2-Methyl-hexen-4-säure (2-MHS) im Sumpf der Kolonne K-1.

Zur Induktion der Phasentrennung in der Waschkolonne K-2 kann statt Frischwasser auch das in der Reaktion anfallende Prozesswasser verwendet werden. Dadurch wird ein erhöhter Abwasserstrom vermieden. Besonders vorteilhaft ist jedoch die Zuführung eines Teilstroms der wässrigen Katalysatorlösung: der ohnehin gelöste Katalsator verbleibt in der wässrigen Phase, die rezykliert wird - geht also nicht verloren, verbessert die Löslichkeit der wieder zu gewinnenden Edukte und erhöht die Dichte der wässrigen Phase, was eine gute und schnelle Phasentrennung begünstigt. Die Massenverhältnisse zwischen Wasser bzw. wässriger Katalysatorlösung und Ausschleusungsstrom 7 betragen 0,5:1-5:1, bevorzugt 1:1-3:1 und besonders bevorzugt 1,1:1-1,5:1. Die Phasentrennung erfolgt bei Temperaturen von 0-60 °C, bevorzugt bei 5-40 °C und besonders bevorzugt bei 10-35°C.

Es wurde gefunden, dass die Wertstoffmenge des Prozesses erhöht werden kann, wenn vor der Phasentrennung das Reaktionsgemisch aus Wasser und ausgeschleustem Reaktionsstrom für mindestens 2 Minuten, bevorzugt mindestens 5 Minuten, besonders bevorzugt mindestens 10 Minuten auf mindestens 70°C erwärmt, bevorzugt auf mindestens 90°C erwärmt wird. Durch die Erwärmung wird das im Reaktionsstrom enthaltene hydrophobe Tetramethylglycolid in den hydrophileren 2-Hydroxyisobuttersäure-1-carboxy-1-methylethylester überführt. Somit verbleibt der hydrophilere 2-Hydroxyisobuttersäure-1-carboxy-1-methylethylester überwiegend in der wässrigen Phase und kann in den Prozess zurückgeführt werden.

Die Rückführung der wässrigen Zielproduktlösung kann sowohl in den Reaktor R-1 als auch in die Destillationskolonne K-1 erfolgen. Bevorzugt ist die Rückführung in letztere.

Zur Auftrennung von Nebenprodukten und Additiven vom Edukt nach dem Ausschleusungspunkt können auch andere Trennverfahren wie Destillation, Kristallisation oder Adsorption angewandt werden. Diese Verfahren sind in gängigen Lehrbüchern der Verfahrenstechnik beschrieben wie z.B. Stichlmair, J. 2010. Distillation, 3. Processes, Ullmann's Encyclopedia of Industrial Chemistry, Published Online: 15.04.2010; Mullin, J. W. 2003, Crystallization and Precipitation, Ullmann's Encyclopedia of Industrial Chemistry, Published Online: 15.01.2003 oder Bart, H.-J. and von Gemmingen, U. 2005, Adsorption, Ullmann's Encyclopedia of Industrial Chemistry. Published Online: 15.01.2005. Bei diesen alternativen Trennverfahren ist in der Regel die Einhaltung einer Induktionsperiode nicht notwendig, allerdings ist ihre Durchführung apparativ und energetisch aufwendiger.

Die Dehydratisierungsreaktion selbst kann in CSTRs oder Schlaufenreaktoren ablaufen, wobei das Zielprodukt aufgrund des im Vergleich zum Edukt niedrigeren Siedepunktes destillativ aus der Reaktionsmischung entfernt wird. Die Reaktion wird bei Drücken von 0,01-1 bar, bevorzugt bei 0,05-0,5 bar, besonders bevorzugt 0,1-0,3 bar und Temperaturen von 30-300 °C, bevorzugt bei 80-250 °C, besonders bevorzugt 180-230 °C durchgeführt.

Als Katalysatoren eignen sich z.B. die im Stand der Technik zitierten Verbindungen, bevorzugt werden Alkalihydroxide eingesetzt. Es werden zweckmäßigerweise 0,1-5 mol, bevorzugt 0,3-3 mol und besonders bevorzugt 0,5-1,5 mol Katalysator bezogen auf die Mol-Äquivalente an alpha-substituierte Carbonsäuren, die pro h dem Reaktor zugeführt werden, verwendet.

Folgende Beispiele sollen die Erfindung erläutern, aber in keiner Weise beschränken.

### Vergleichsbeispiel 1

In einem Reaktor werden 32,4 kg einer Schmelze bestehend 83,6 Gew% 2-HIBS, 12 Gew% Tetramethylglykolid, 3,9 Gew% 2-Hydroxyisobuttersäure-1-carboxy-1-methylethylester und 0,5 Gew% 2-Methoxyisobuttersäure vorgelegt. Die Schmelze ist stabilisiert mit je 1000 ppm Hydrochinonmonomethylether (HQME) und 2,4-Dimethyl-6-tert-butylphenol (Topanol A). Der Schmelze im Reaktor werden 13,75 kg einer 85%igen KOH zugegeben, wobei der Reaktorinhalt bei Zugabe des Katalysators auf ca. 150 °C gehalten wird. Nach Zugabe des Katalysators wird der Reaktorinhalt auf 215 °C durch Umpumpen über einen Fallfilmwärmetauscher erhitzt und ein Vakuum von 150 mbar angelegt. Der Fallfilmwärmetauscher wird auf der Mantelseite mittels eines Wärmeträgeröls (Marlotherm SH) beheizt. In die Rohre des Fallfilmwärmetauschers sind Turbulatoren der Fa. Calgavin zur Verbesserung des Wärmeübergangs eingebracht. Im stationären Betrieb wird das Wärmeträgeröl zum Erreichen der Reaktionstemperatur auf 225°C erhitzt. Dem Reaktor wird dann ein Feedstrom von 11,2 kg/h mit obiger Zusammensetzung zugeführt. Der Brüdenabstrom des Reaktors wird in die nachgeschaltete Kolonne geleitet, in der ein Vakuum von 80 mbar anliegt. Die Reaktionsprodukte MAS und Wasser werden als Kopfprodukt abgezogen. Zur Erzielung einer besseren Trennleistung werden 20 kg/h des erhaltenen Destillats wieder als Rücklauf auf die Kolonne gegeben. Zudem wird der Kondensator zur Vermeidung von Polymerisation mit 200 ml/h einer methanolischen Lösung von 10 Gew% HQME und 5 Gew% Topanol A besprüht. Am Sumpf der Kolonne werden nicht umgesetzte Produkte, Polymerisationsinhibitoren und hochsiedende Nebenprodukte abgetrennt und zurück in den Reaktor gefahren. Der erhaltene Destillatstrom an der Kolonne betrug 11,4 kg/h. Ein kleiner Teil des Kopfprodukts geht unkondensiert ins Vakuumsystem. Zur Vermeidung der Anreicherung von polymeren Nebenprodukten im Reaktor wird 12 h nach Reaktionsbeginn kontinuierlich ein Strom von 200 g/h aus dem Reaktor ausgeschleust. Zur Substitution des Verlusts an Kalium werden 300 g/h KOH als 17%ige Lösung in H₂O nachdosiert. Die Umsetzung wird über einen Zeitraum von 4 Wochen betrieben. Nach 4 Wochen wurde der Versuch abgebrochen, da der Rückführstrom vom Sumpf der Kolonne in den Reaktor stark zugenommen hatte.

Der Feedstrom, das Destillat der Kolonne sowie der Rückführstrom der Kolonne wurden regelmäßig beprobt und analysiert. Dabei werden 2-HIBS, Tetramethylglykolid, 2-Hydroxyisobuttersäure-1-carboxy-1-methylethylester und Methacrylsäure mittels HPLC gemessen, während alle anderen Komponenten H₂O, Methanol, 2-Methoxyisobuttersäure sowie HQME und Topanol A per GC analysiert werden. Die Komponenten 2-HIBS, Tetramethylglykolid und 2-Hydroxyisobuttersäure-1-carboxy-1-methyethylester werden dabei in dem Summenparameter w_HIBS_eq als molare Äqivalente von 2-HIBS zusammengefasst. Das während der Umsetzung erhaltene Destillat bestand im Mittel aus 19,0 Gew% H₂O, 80,4 Gew% Methacrylsäure sowie 0,2 Gew% 2-Methoxyisobuttersäure und 0,4 Gew% Methanol.

Bei der Reaktion wird dabei ein Umsatzgrad pro Reaktordurchgang von w_HIBS_eq = 67% bei einer Selektivität bzgl. Methacrylsäure von 97,4% erreicht. Als Nebenprodukte entstehen vornehmlich cis-und trans 2-Methyl-hexen-4-säure (2-MHS).

Die Analysenergebnisse des Recyclestroms vom Kolonnensumpf zurück in den Reaktor für die Versuchzeiten von 100, 250, 390 und 650 h sind in Tab. 1 in Gew% zusammengefasst.

**Tab. 1: Analyse des Recyclestroms**

| Komponente im Kolonnensumpf | 100 h | 250 h | 390 h | 650 h |
|---|---|---|---|---|
| Gew% | Sumpf A | Sumpf B | Sumpf C | Sumpf D |
| 2-HIBS | 74,0 | 63,1 | 58,3 | 34,4 |
| Tetramethylglykolid | 13,0 | 9,1 | 4,3 | 4,0 |
| 2-Hydroxyisobuttersäure-1-carboxy-1-methylethylester | 0,75 | 0,6 | 0,5 | 0,2 |
| 2-Methoxyisobuttersäure | 0,5 | 0,5 | 0,5 | 0,4 |
| 2-MHS | 8,0 | 19,0 | 27,4 | 50,9 |
| ∑ (HQME + Topanol A) | 1,6 | 3,5 | 3,9 | 6,3 |
| Phasentrennung mit Wasser | nein | ja | ja | ja |
| Phasentrennung mit KOH | nein | ja | ja | ja |

Die Ergebnisse bestätigen, dass 2-Methoxyisobuttersäure abreagiert, da sich kein Anreicherungseffekt zeigt. Der Gesamtumsatzgrad von 2-Methoxyisobuttersäure über die gesamte Prozesseinheit erreicht 60%. Auch dimeren Verbindungen von 2-HIBS (2-Hydroxyisobuttersäure-1-carboxy-1-methyethylester und Tetramethyl-glykolid) reichern sich nicht an, sondern reagieren ab. Aufgrund der hohen erreichten Selektivität zu Methacrylsäure muss davon ausgegangen werden, dass auch diese Verbindungen zumindest zum großen Teil zu Methacrylsäure abreagieren. Nebenprodukte wie 2-MHS als auch die Polymerisationsinhibitoren akkumulieren über die Betriebsdauer dagegen beständig, ohne auf ein stationäres Niveau zu kommen. Ferner ist zu sehen, dass an aus den jeweiligen Kolonnensümpfen entnommenen Proben erst ab einer bestimmten Konzentration an Nebenprodukten eine Phasentrennung induziert werden kann. Die Zusammensetzung der Phasen wird als Beispiel an Sumpf B in Tab. 2 dargestellt.

**Tab. 2: Massenanteile beider Phasen und Zusammensetzung in Gew%**

| | Sumpf B plus Wasser | | Sumpf B plus KOH | |
|---|---|---|---|---|
| Komponente im Kolonnensumpf | wässrige Phase | organische Phase | wässrige Phase | organische Phase |
| Massenanteil in % | 83 | 17 | 89 | 11 |
| Davon | | | | |
| H₂O | 73 | 36 | 52 | 32 |
| Kalium als KOH | - | - | 9,4 | 5,7 |
| w_HIBS_eq | 25 | 20 | 37 | 21 |
| 2-MHS | 1,7 | 30 | 6,3 | 28 |
| ∑ (HQME + Topanol A) | 0,1 | 6,5 | 0,7 | 8,6 |

### Beispiel 1

Als erfindungsgemäßes Beispiel 1 wurde Vergleichsbeispiel 1 im Wesentlichen wiederholt, wobei an der Versuchsanordnung und Durchführung folgende Veränderungen vorgenommen wurden: Dem Rückführstrom aus dem Sumpf der Kolonne wird bei Überschreitung einer Gesamtkonzentration von 20 Gew% an 2-MHS sowie den Inhibitoren HQME und Topanol A - hier nach einer Versuchsdauer von 200 h - ein kleiner Teilstrom von 0,4 kg/h entnommen. Dieser Teilstrom wird mit 0,5 kg/h Wasser gemischt und einem auf 15 °C temperierten Verweiler zur Phasentrennung zugeführt. Die wässrige Phase wird zurückgeführt auf die Mitte der dem Reaktor nachgeschalteten Kolonne. Die organische Phase wird ausgeschleust und verworfen. Der Zustrom an Polymerisationshibitorlösung betrug 300 ml/h. Der Versuch wird über einen Zeitraum von 6 Wochen betrieben. Der erhaltene Destillatstrom an der Kolonne betrug 11,9 kg/h. Das während er Umsetzung erhaltene Destillat bestand im Mittel aus 22,0 Gew% H₂O, 77,0 Gew% Methacrylsäure sowie 0,1 Gew% 2-Methoxyisobuttersäure und 0,9 Gew% Methanol.

Bei der Reaktion wird dabei ein Umsatzgrad pro Reaktordurchgang von w_HIBS_eq = 64% bei einer Selektivität bzgl. Methacrylsäure von 97,1 % erreicht. Als Nebenprodukte entsteht vornehmlich 2-MHS.

Die Analysenergebnisse des Recyclestrom vom Kolonnensumpf zurück in den Reaktor für die Versuchzeiten von 190, 360, 700 und 950 h sind in Tab. 3 in Gew% zusammengefasst.

**Tab. 3: Analysen des Recyclestroms**

| **Komponente im Kolonnensumpf (Gew%)** | **190 h** | **360 h** | **700 h** | **950 h** |
|---|---|---|---|---|
| 2-HIBS | 65 | 55 | 55 | 55 |
| Tetramethylglykolid | 11 | 9,5 | 9,0 | 8,5 |
| 2-Hydroxyisobuttersäure-1-carboxy-1-methyethylester | 0,7 | 0,5 | 0,6 | 0,8 |
| 2-Methoxyisobuttersäure | 0,6 | 0,7 | 0,6 | 0,6 |
| 2-MHS | 15,0 | 21,6 | 20,4 | 21,2 |
| ∑ (HQME + Topanol A) | 4,5 | 6,5 | 6,8 | 7,7 |

Die durchschnittlichen Analysenergebnisse des Phasenseparators für die wässrige und die organische Phase für den Betriebszeitraum 200-1000 h sind in Tab. 4 zusammengefasst.

**Tab. 4: Massenanteile beider Phasen und Zusammensetzung in Gew%**

| **Komponente im Kolonnensumpf** | **Beispiel 1 wässrige Phase** | **Beispiel 1 organische Phase** |
|---|---|---|
| Massenanteil in % | 82 | 18 |
| Davon | | |
| H₂O | 61,4 | 35,5 |
| w_HIBS_eq | 30,4 | 24,3 |
| 2-MHS | 6,0 | 29,3 |
| 2-Methoxyisobuttersäure | 0,7 | 1,0 |
| ∑ (HQME + Topanol A) | 1,5 | 9,9 |

### Beispiel 2

Das erfindungsgemäße Beispiel 1 wurde im Wesentlichen wiederholt, wobei an der Versuchsanordnung und Durchführung folgende Veränderungen vorgenommen wurden: Der Fallfilmreaktor wurde durch einen Plattenwärmetauscher ersetzt. Im Gegensatz zu dem Fallfilmverdampfer wird durch Einstellen eines Überdrucks von 3 bara mit Hilfe eines Druckhalteventils gewährleistet, dass der Plattenwärmetauscher mit Flüssigkeit gefüllt ist. Hinter dem Plattenwärmetauscher wird eine Temperatur von 215 °C eigestellt. Hinter dem Druckhalteventil wird der Umlaufstrom auf 150 mbara entspannt.

Der Zustrom an Polymerisationshibitorlösung zur Kolonne betrug 300 ml/h. Der Versuch wird über einen Zeitraum von 45 Tagen betrieben. Der erhaltene Destillatstrom an der Kolonne betrug 11,8 kg/h. Das während er Umsetzung erhaltene Destillat bestand im Mittel aus 22,5 Gew% H₂O, 77,3 Gew% Methacrylsäure, 0,5% Tetramethylgycolid sowie 0,1 Gew% 2-Methoxyisobuttersäure und 0,9 Gew% Methanol.

Bei der Reaktion wird dabei ein Umsatzgrad pro Reaktordurchgang von w_HIBS_eq = 65,5% bei einer Selektivität bzgl. Methacrylsäure von 97,8 % erreicht. Als Nebenprodukte entsteht vornehmlich 2-MHS.

Die Analysenergebnisse des Recyclestrom vom Kolonnensumpf zurück in den Reaktor für die Versuchszeiten von 950 und 1090 h sind in Tab. 5 in Gew.% zusammengefasst.

**Tab. 5: Analysen den Recyclestroms zu Beispiel 2 in Gew%**

| **Komponente im Kolonnensumpf** | **950 h** | **1090 h** |
|---|---|---|
| 2-HIBS | 45,0 | 49,0 |
| Tetramethyl-glykolid | 19,4 | 20,5 |
| 2-Hydroxyisobuttersäure-1-carboxy-1-methyethylester | 0,6 | 1,0 |
| 2-Methoxyisobuttersäure | | |
| 2-MHS | 17,6 | 13,5 |
| ∑ (HQME + Topanol A) | 4,6 | 4,2 |

Die durchschnittlichen Analysenergebnisse des Phasenseparators für die wässrige und die organische Phase für den Betriebszeitraum 950-1090h sind in Tab. 6 zusammengefasst.

**Tab. 6: Massenanteile und Zusammensetzung beider Phasen in Gew%**

| **Komponente im Kolonnensumpf** | **Beispiel 2 wässrige Phase** | **Beispiel 2 organische Phase** |
|---|---|---|
| Massenanteil in % | 76 | 22 |
| davon | | |
| H₂O | 63,3 | 22,6 |
| w_HIBS_eq | 30,3 | 31,5 |
| 2-MHS | 5,1 | 37,4 |
| 2-Methoxyisobuttersäure | 0,4 | 0,3 |
| ∑ (HQME + Topanol A) | 0,6 | 8,3 |

Die erfindungsgemäßen Beispiel 1 und 2 zeigen, dass durch Entnahme eines Teilstroms der Sumpfrückführung sowie der extraktiven Behandlung des ausgeschleusten Teilstroms mit Wasser und nachfolgende Rückführung der edukt-haltigen wässrigen Phase ein langfristig stabiler Betrieb einer MAS-Produktion aus 2-HIBS möglich ist und Nebenprodukte sowie Polymerisationsinhibitoren gezielt und selektiv aus dem Prozess entfernt werden können.

### Beispiel 3

Das erfindungsgemäße Beispiel 1 wurde im Wesentlichen wiederholt, wobei an der Versuchsanordnung und Durchführung folgende Veränderungen vorgenommen wurden:
Der zu Extraktion der MHS eingesetzte Wasserstrom wurde auf 0,6 kg/h eingestellt. Der Ausschleusestroms des Kolonnensumpfes mit 0,4 kg/h und der Wasserstrom zur Extraktion werden gemischt und vor der Aufgabe auf den Verweiler zur Phasentrennung für 10 min in einem beheizten Rohr auf 90°C erhitzt. Anschließend wird der Strom auf 40°C abgekühlt, die Temperatur im Phasentrenner beträgt ebenfalls 40°C.

Die durchschnittlichen Analysenergebnisse des Phasenseparators für die wässrige und die organische Phase für den Betriebszeitraum nach ca. 900h sind in Tab. 7 zusammengefasst.

**Tab. 7: Massenanteile und Zusammensetzung beider Phasen in Gew%**

| **Komponente im Kolonnensumpf** | **Beispiel 3 wässrige Phase** | **Beispiel 3 organische Phase** |
|---|---|---|
| Massenanteil in % | 77 | 23 |
| Davon | | |
| H₂O | 68,0 | 39,0 |
| w_HIBS_eq | 30,5 | 14,2 |
| 2-MHS | 0,5 | 37,9 |
| 2-Methoxyisobuttersäure | 0,3 | 0,6 |
| ∑ (HQME + Topanol A) | 0,5 | 8,1 |

Durch das kurzzeitige Erhitzen des mit Wasser gemischten Ausschleusestroms kann der Anteil an Wertstoff (w_HIBS_eq) in der ausgeschleusten organischen Phase und somit der Wertstoffverlust nochmals deutlich reduziert werden.

### Versuchsbedingungen:

### 1. HPLC

| **Komponente** | **Bezeichnung** |
|---|---|
| Pumpe | Dionex HPLC Pump 680 |
| Säulenofen | Thermostated column compartment TCC-100 |
| UV-Detektor | Ultimate 3000 Variable wavelength detector |
| HPLC-Säule | Zorbax SB-Aq Rapid Solution 4.6x150 mm ; 3.5 µm |
| Elutionsmittel | Acetonitril LiChrosolv (Fa. Merck) |
| | Kaliumdihydrogenphosphat-Lösung (0,02 mol/l) |

Temperatur 55°C

### 2. GC

| **Komponente** | **Bezeichnung** |
|---|---|
| GC-Gerät | CP-3800 Gaschromatograph |
| Injektor | 1079 PTV |
| Säule | AT-WAX (Alltech); Länge: 30 m; ID: 0,53 mm; Film: 1,2 µm |
| Detektor | WLD |
| Trägergas | Helium |

Temperaturprogramm: 40°C bis 240°C mit 20 K/min Steigerung

## Patentansprüche

1. Verfahren zur Dehydratisierung von alpha-substituierten Carbonsäuren **dadurch gekennzeichnet, dass** kontinuierlich zumindest ein Teil des Reaktionsstroms aus dem Reaktionskreislauf ausgeschleust, Nebenprodukte oder überschüssige Additive vom nicht umgesetzten Ausgangsprodukt abgetrennt werden und letzteres in den Reaktionskreislauf zurückgeführt wird.

2. Verfahren nach Anspruch 1 **dadurch gekennzeichnet, dass** die Abtrennung der Nebenprodukte vom Ausgangsprodukt durch Phasentrennung erfolgt.

3. Verfahren nach Anspruch 2 **dadurch gekennzeichnet, dass** die Phasentrennung durch Wasser oder wässrige Katalysatorlösung induziert wird.

4. Verfahren nach Anspruch 3 **dadurch gekennzeichnet, dass** zur Induktion der Phasentrennung das im Prozess entstandene Reaktionswasser verwendet wird.

5. Verfahren nach Anspruch 3 **dadurch gekennzeichnet, dass** die Phasentrennung bei 0-60 °C durchgeführt wird.

6. Verfahren nach Anspruch 5 **dadurch gekennzeichnet, dass** vor der Phasentrennung das Reaktionsgemisch aus Wasser und ausgeschleustem Reaktionsstrom für mindestens 2 Minuten auf mindestens 70°C erwärmt wird.

7. Verfahren nach Anspruch 1 **dadurch gekennzeichnet, dass** die Abtrennung der Nebenprodukte vom Ausgangsprodukt durch Destillation erfolgt.

8. Verfahren nach Anspruch 1 **dadurch gekennzeichnet, dass** die Abtrennung der Nebenprodukte vom Ausgangsprodukt durch Kristallisation erfolgt.

9. Verfahren nach Anspruch 1 **dadurch gekennzeichnet, dass** die Abtrennung der Nebenprodukte vom Ausgangsprodukt durch Adsorption erfolgt.

10. Verfahren nach Anspruch 1 **dadurch gekennzeichnet, dass** die Rückführung des Ausgangsproduktes in die Destillationskolonne erfolgt, in der das Produkt über Kopf abgezogen wird.

11. Verfahren nach Anspruch 1 **dadurch gekennzeichnet, dass** die Rückführung des Ausgangsproduktes in den Reaktorkreislauf erfolgt.

12. Verfahren nach Anspruch 1 **dadurch gekennzeichnet, dass** die Ausschleusung zumindest eines Teils des Reaktionsstroms aus dem Sumpf der Destillationskolonne erfolgt, in der das Produkt über Kopf abgezogen wird.

13. Verfahren nach Anspruch 1 **dadurch gekennzeichnet, dass** die Ausschleusung zumindest eines Teils des Reaktionsstroms aus dem Reaktorkreislauf erfolgt.

14. Verfahren nach Anspruch 1 **dadurch gekennzeichnet, dass** die Ausschleusung zumindest eines Teils des Reaktionsstroms erst nach einer Induktionsperiode erfolgt.

15. Verfahren nach Anspruch 1 zur Dehydratisierung von 2-Hydroxyisobuttersäure.

## Claims

1. Process for dehydrating alpha-substituted carboxylic acids, **characterized in that** at least part of the reaction stream is continuously discharged from the reaction circuit, by-products or excess additives are separated off from the unreacted starting material and the latter is reintroduced into the reaction circuit.

2. Process according to Claim 1, **characterized in that** the by-products are separated off from the starting material by phase separation.

3. Process according to Claim 2, **characterized in that** phase separation is induced by means of water or aqueous catalyst solution.

4. Process according to Claim 3, **characterized in that** the water of reaction formed in the process is used to induce phase separation.

5. Process according to Claim 3, **characterized in that** the phase separation is carried out at 0-60°C.

6. Process according to Claim 5, **characterized in that** the reaction mixture of water and discharged reaction stream is heated for at least 2 minutes to at least 70°C before the phase separation.

7. Process according to Claim 1, **characterized in that** the by-products are separated off from the starting material by distillation.

8. Process according to Claim 1, **characterized in that** the by-products are separated off from the starting material by crystallization.

9. Process according to Claim 1, **characterized in that** the by-products are separated off from the starting material by adsorption.

10. Process according to Claim 1, **characterized in that** the starting material is recirculated to the distillation column in which the product is taken off at the top.

11. Process according to Claim 1, **characterized in that** the starting material is recirculated to the reactor circuit.

12. Process according to Claim 1, **characterized in that** the discharge of at least part of the reaction stream is effected from the bottoms from the distillation column in which the product is taken off at the top.

13. Process according to Claim 1, **characterized in that** the discharge of at least part of the reaction stream is effected from the reactor circuit.

14. Process according to Claim 1, **characterized in that** the discharge of at least part of the reaction stream is effected only after an induction period.

15. Process according to Claim 1 for dehydrating 2-hydroxyisobutyric acid.

## Revendications

1. Procédé pour la déshydratation d'acides carboxyliques substitués en position alpha, **caractérisé en ce qu'**on soutire en continu au moins une partie du flux de réaction du circuit de réaction, on sépare les produits secondaires ou les additifs en excès du produit de départ non transformé et on recycle ce dernier dans le circuit de réaction.

2. Procédé selon la revendication 1, **caractérisé en ce que** la séparation des produits secondaires du produit de départ a lieu par séparation des phases.

3. Procédé selon la revendication 2, **caractérisé en ce que** la séparation des phases est induite par de l'eau ou par une solution aqueuse de catalyseur.

4. Procédé selon la revendication 3, **caractérisé en ce que** l'eau de réaction formée dans le procédé est utilisée pour l'induction de la séparation des phases.

5. Procédé selon la revendication 3, **caractérisé en ce que** la séparation des phases est réalisée à 0-60°C.

6. Procédé selon la revendication 5, **caractérisé en ce que** le mélange réactionnel constitué par de l'eau et le flux de réaction soutiré est chauffé pendant au moins 2 minutes à au moins 70°C avant la séparation des phases.

7. Procédé selon la revendication 1, **caractérisé en ce que** la séparation des produits secondaires du produit de départ a lieu par distillation.

8. Procédé selon la revendication 1, **caractérisé en ce que** la séparation des produits secondaires du produit de départ a lieu par cristallisation.

9. Procédé selon la revendication 1, **caractérisé en ce que** la séparation des produits secondaires du produit de départ a lieu par adsorption.

10. Procédé selon la revendication 1, **caractérisé en ce que** le recyclage du produit de départ a lieu dans la colonne de distillation dans laquelle le produit est soutiré via la tête.

11. Procédé selon la revendication 1, **caractérisé en ce que** le recyclage du produit de départ a lieu dans le circuit du réacteur.

12. Procédé selon la revendication 1, **caractérisé en ce que** le soutirage d'au moins une partie du flux de réaction a lieu à partir du fond de la colonne de distillation dans laquelle le produit est soutiré via la tête.

13. Procédé selon la revendication 1, **caractérisé en ce que** le soutirage d'au moins une partie du flux de réaction a lieu à partir du circuit du réacteur.

14. Procédé selon la revendication 1, **caractérisé en ce que** le soutirage d'au moins une partie du flux de réaction n'a lieu qu'après une période d'induction.

15. Procédé selon la revendication 1 pour la déshydratation de l'acide 2-hydroxyisobutyrique.
